# EUROPÄISCHE PATENTANMELDUNG

(11) **EP 1 779 835 A1**
(43) Veröffentlichungstag der Anmeldung: **02.05.2007**
(21) Anmeldenummer: 06118924.7
(22) Anmeldetag: 15.08.2006
(51) Int. Cl.: A61K 8/04, A61K 8/34, A61K 8/92, A61Q 5/00, A61Q 19/00

(54) **Emulsion enthaltend 1,2-Alkandiole und polare Ölkomponenten**

(30) Priorität: 25.10.2005 DE 102005051862
(71) Anmelder: Beiersdorf AG, 20253 Hamburg (DE)
(72) Erfinder: Bleckmann, Andreas, 22926, Ahrensburg (DE); Clausen, Andreas, 20146, Hamburg (DE); Meiring, Uta, 21077, Hamburg (DE); Behrens, Svea, 21109, Hamburg (DE); Nielsen, Jens, 24558, Henstedt-Ulzburg (DE)

(57) **Zusammenfassung**

Die vorliegende Erfindung betrifft Emulsionen als kosmetische Zubereitungen mit einem Anteil an 1,2-Alkandiolen und polaren Ölkomponenten. Die erfindungsgemäßen Formulierungen weisen gute sensorische Eigenschaften auf.

## Beschreibung

Die vorliegende Erfindung betrifft Emulsionen als kosmetische Zubereitungen mit einem Anteil an 1, 2-Alkandiolen und polaren Ölkomponenten. Die erfindungsgemäßen Formulierungen weisen gute sensorische Eigenschaften auf, die u. a. in verbesserten in-vivo Wirksamkeiten resultieren.

Die Haut ist unser größtes Organ und gleichzeitig Spiegelbild unserer Seele. Kein anderes Organ ist äußeren Einflüssen so stark ausgesetzt. Hitze, Kälte oder Sonneneinstrahlung, falsche oder übertriebene Hautreinigung - das alles belastet die Haut.

Jeden Tag verdunstet dabei über die Hautoberfläche Wasser. Innere und äußere Faktoren beeinflussen den Grad der Austrocknung zusätzlich, wie hormonelle Einflüsse, die biologische Hautalterung, Krankheiten sowie Ernährung, Licht, Umwelt und Klima.

Normaler Haut und insbesondere trockener Haut müssen daher Feuchtigkeit, Lipide und ggf. Wirkstoffe zugeführt werden. Gesunde Haut hat verschiedene Möglichkeiten, den Wasserverlust in Grenzen zu halten. Sie verfügt über so genannte Feuchthaltefaktoren, nämlich Salze, verschiedene organische Säuren und Harnstoff (Urea). Diese Faktoren übernehmen die Aufgabe, Feuchtigkeit zu binden und in der Haut festzuhalten. Genauso wichtig sind die Hautfette, Lipide. Nur mit Hilfe dieses intakten Schutzwalls ist es der oberen Hautschicht möglich, den Wassergehalt zu regulieren und zusätzlich dafür zu sorgen, dass Infektionserreger wie Bakterien, Vieren oder Pilze sowie andere schädigende Stoffe nicht ungehindert eindringen können.

Trockene Haut ist empfindlich und braucht vor allem Pflege. Kosmetische Zubereitungen wie Cremes und Lotionen haben in erster Linie die Aufgabe, den Fett- und Feuchtigkeitsbedarf der Haut nachhaltig auszugleichen und damit das natürliche Gleichgewicht in der Haut wieder herzustellen. Deshalb ist es wichtig, Präparate zu verwenden, die hautverwandte Lipide, Feuchthaltemittel und pflegende Wirkstoffe gleichermaßen enthalten. Zusätzlich braucht ein Pflegeprodukt auch Wirkstoffe, die in der Lage sind, Wasser langfristig in der Haut zu binden.

Lipide bezeichnen Fette und fettähnliche Stoffe. Für die Kosmetik sind sie vor allem als weichmachende ("emollient") Inhaltsstoffe von Bedeutung und als hauteigene Lipide der Hornschicht, die zwischen den Hornzellen lagern. Sie befähigen die Haut zur Speicherung von Feuchtigkeit. Neben dem pflegenden Aspekt werden Lipide den kosmetischen Zubereitungen zugesetzt um eine bessere Verteilbarkeit auf der Haut zu gewährleisten und um die sensorischen Eigenschaften der Zubereitungen zu verbessern.

Im Rahmen der vorliegenden Offenbarung wird als Oberbegriff für Fette, Öle, Wachse und dergleichen gelegentlich der Ausdruck "Lipide" verwendet, wie dem Fachmanne durchaus geläufig ist. Auch werden die Begriffe "Ölphase" und "Lipidphase" synonym angewandt.

Öle und Fette unterscheiden sich unter anderem in ihrer Polarität, welche schwierig zu definieren ist. Es wurde bereits vorgeschlagen, die Grenzflächenspannung gegenüber Wasser als Maß für den Polaritätsindex eines Öls bzw. einer Ölphase anzunehmen. Dabei gilt, dass die Polarität der betreffenden Ölphase umso größer ist, je niedriger die Grenzflächenspannung zwischen dieser Ölphase und Wasser ist. Erfindungsgemäß wird die Grenzflächenspannung als ein mögliches Maß für die Polarität einer gegebenen Ölkomponente angesehen.

Die Grenzflächenspannung ist diejenige Kraft, die an einer gedachten, in der Grenzfläche zwischen zwei Phasen befindlichen Linie der Länge von einem Meter wirkt. Die physikalische Einheit für diese Grenzflächenspannung errechnet sich klassisch nach der Beziehung Kraft/Länge und wird gewöhnlich in mN/m (Millinewton geteilt durch Meter) wiedergegeben. Sie hat positives Vorzeichen, wenn sie das Bestreben hat, die Grenzfläche zu verkleinern. Im umgekehrten Falle hat sie negatives Vorzeichen. Als polar im Sinne der vorliegenden Idee werden Lipide angesehen, deren Grenzflächenspannung gegen Wasser weniger als 20 mN/m beträgt, als unpolar solche, deren Grenzflächenspannung gegen Wasser mehr als 30 mN/m beträgt. Lipide mit einer Grenzflächenspannung gegen Wasser zwischen 20 und 30 mN/m werden im allgemeinen als mittelpolar bezeichnet.

EP 655904 beschreibt allgemein die Verwendung von Alkandiolen mit 5 bis 10 C-Atomen in kosmetischen Produkten und insbesondere deren hautbefeuchtende Wirkung.

Die DE 10341179 offenbart Deodorantzusammensetzungen mit einer Kombination aus Alkan-1,2-diolen und α- und/oder β-Hydroxysäuren. Auch ist die antibakterielle Wirkung von Alkan-1,2-diolen bekannt. So beschreibt die JP 20022003330 die antibakterielle Wirkung einer Kombination von 1,2-Alkandiolen und Ascorbinsäureestern. Die WO 03/000220 beschreibt die Verwendung von 1,2-Decandiol gegen Körpergeruch verursachende Bakterien. Ebenso beschreibt die US 6,123,953 die Verwendung von Alkan-1,2-diolen zur Inaktivierung von Mikroorganismen auf der Haut durch den Einsatz von 1,2-Octandiol.

Als kosmetische oder medizinische Zubereitungen sind oftmals Emulsionen, hier insbesondere W/O-, O/W- oder W/O/W-Emulsionen, im Einsatz. Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten (W/O) in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Natürlich ist dem Fachmann eine Vielzahl von Möglichkeiten bekannt, stabile Emulsionszubereitungen zur kosmetischen oder dermatologischen Anwendung zu formulieren, beispielsweise in Form von Cremes und Salben, die im Bereich von Raum- bis Hauttemperatur streichfähig sind, oder als Lotionen und Milche, die in diesem Temperaturbereich eher fließfähig sind.

Aus DE 1001921 sind eine Vielzahl von Lipiden bekannt, die als Zusatz zu W/O-Emulsionen bessere sensorische Eigenschaften, wie beispielsweise die Verteilbarkeit auf der Haut oder das Einzugsvermögen in die Haut, aufweisen.

Problem bei allen Zubereitungen ist es aber stabile Emulsionen mit einer überwiegend polaren Ölphase herzustellen ohne Einbussen hinsichtlich der Sensorik einzugehen.

Im Stand der Technik wird dieses Problem der Grenzflächenaktivität der Lipide durch Zusatz von geeigneten Verdickersystemen versucht zu lösen, was wiederum die erwähnten Nachteile hinsichtlich der sensorischen Eigenschaften der kosmetischen Zubereitung zur Folge hat.

So wird z. B. in DE 198 55 153, DE 199 24 277, DE 100 48 125, DE 100 48 366, DE 100 48 427, DE 100 48 429 sowie in DE 100 48 683 erwähnt, dass Zubereitungen in Kombination mit stark polaren Ölen - wie z. B. die in den handelsüblichen Produkten sonst häufig verwendeten Pflanzenöle - den Nachteil besitzen, dass sie instabil sind bzw. auf einen engen Anwendungsbereich oder eine sehr begrenzte Einsatzstoffauswahl begrenzt sind.

Aufgabe der vorliegenden Erfindung ist es daher, stabile Emulsionen und insbesondere kosmetische Zubereitungen bereit zu stellen, die überwiegend polare Ölkomponenten enthalten.

Gelöst werden die Aufgaben durch eine Emulsion nach Anspruch 1. In den Unteransprüchen sind bevorzugte Ausführungsformen der Emulsion offenbart. Die Erfindung umfasst darüber hinaus auch die Verwendung der Emulsionen.

Es war überraschend und für den Fachmann nicht vorauszusehen, dass eine Emulsion enthaltend über 40 Gew.-% ein oder mehrerer polarer und/oder mittelpolarer Lipide, bezogen auf das Gesamtgewicht der Lipidphase der Emulsion, mit einer Grenzflächenspannung gegen Wasser von weniger als 30 mN/m und ein oder mehrere 1, 2 - Alkandiole mit 4 bis 8 C-Atomen und einem lg p_{ow} im Bereich von - 1 bis + 3, insbesondere im Bereich von -1 bis +1, die Aufgaben löst.

Es ist erfindungsgemäß von Vorteil, wenn die Emulsionen als kosmetische Zubereitungen verwendet werden. Vorteilhaft ist zudem, dass die erfindungsgemäßen Zubereitungen, in denen die erfindungsgemäß verwendete Wirkstoffkombination enthalten ist, dadurch gekennzeichnet sind, dass als Alkan-1,2-diol 1,2 Hexandiol eingesetzt wird.

Als besonders bevorzugt hat sich darüber hinaus auch die Kombinationen von 1, 2 Hexandiol mit Methylpropandiol sowie auch mit Ethylhexylglycerin gezeigt.

Bevorzugt sind daher Emulsionen umfassend über 40 Gew.-% ein oder mehrerer polarer und/oder mittelpolarer Lipide, bezogen auf das Gesamtgewicht der Lipidphase der Emulsion, mit einer Grenzflächenspannung gegen Wasser von weniger als 30 mN/m und ein oder mehrere 1, 2 - Alkandiole mit 4 bis 8 C-Atomen und einem Ig p_{OW} im Bereich von - 1 bis + 3, insbesondere im Bereich von -1 bis +1, wobei als Alkandiol 1,2 Hexandiol oder die Kombination 1,2 Hexandiol und Methylpropandiol oder 1,2 Hexandiol und Ethylhexylglycerin oder 1,2 Hexandiol, Methylpropandiol und Ethylhexylglycerin gewählt werden.

Ig p_{ow} ist der logarithmische K_{ow}, 1-Octanol-Wasser-Verteilungskoeffizient. Eine Abkürzung für den Verteilungskoeffizienten 1-Octanol/Wasser, das ist das Verhältnis zwischen den Konzentrationen eines Stoffes in den zwei Phasen 1-Octanol (apolar) und Wasser (polar) im Gleichgewicht. P_{ow} steigt mit zunehmender Fettlöslichkeit und sinkender Wasserlöslichkeit. Je größer P_{ow} ist, desto eher kann eine Bioakkumulation in Körperfetten (Membranen, Speicherfett) stattfinden. Bei sehr hohen P_{OW} (Ig P_{OW} > 6,5) wird häufig jedoch eine Abnahme der Bioakkumulationstendenz beobachtet.

Insbesondere sind diejenigen Alkan - 1,2 - diole bevorzugt mit einem lg p_{ow} im Bereich von -1 bis +1, da Alkandiole in diesem bevorzugten lg P_{ow} Bereich eine ausgewogene Verteilung zwischen der Grenzfläche Wasser / Öl aufweisen und somit zur Grenzflächenstabilisierung beitragen.

Polare Öle mit einem Grenzflächenspannung gegen Wasser von weniger als 20 mN/m sind besonders bevorzugt. Hierbei insbesondere solche aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr sowie aus der Gruppe der Wachse Jojobaöl.

Besonders vorteilhafte polare Lipide im Sinne der vorliegenden Idee sind alle nativen Lipide, wie z. B. Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianussöl, Maiskeimöl, Avocadoöl und dergleichen sowie die im folgenden aufgelisteten.

| Handelsname | INCI-Name | Polarität [mN/m] |
|---|---|---|
| Isofol 14 T | Butyl Decanol (+) Hexyl Octanol (+) Hexyl Decanol (+) Butyl Octanol | 19,8 |
| Lipovol MOS-130 | Tridecyl Stearate(+) Tridecyl Trimellitate(+) Dipentaerythrityl Hexacaprylate/Hexacaprate | 19,4 |
| Ricinusoel | | 19,2 |
| Isofol Ester 0604 | | 19,1 |
| Miglyol 840 | Propylene Glycol Dicaprylate/Dicaprate | 18,7 |
| Isofol 12 | Butyl Octanol | 17,4 |
| Tegosoft SH | Stearyl Heptanoate | 17,8 |
| Avocadooel | | 14,5 |
| Cetiol B | Dibutyl Adipate | 14,3 |
| Dermol 488 | PEG 2 Diethylenhexanoate | 10,1 |
| Cosmacol ELI | C12-13 Alkyl Lactate | 8,8 |
| Dermol 489 | Diethylen Glycol Dioctanoate(/ Diisononanoate | 8,6 |
| Cosmacol ETI | Di-C12/13 Alkyl Tartrate | 7,1 |
| Emerest 2384 | Propylene Glycol Monoisostearate | 6,2 |
| Myritol 331 | Cocoglycerides | 5,1 |
| Prisorine 2041 GTIS | Triisostearin | 2,4 |

Neben den als polare Lipide oder Ölkomponenten bezeichneten Stoffe sind aber auch mittelpolare Lipide bevorzugt. Mittelpolare Lipide weisen eine Grenzflächenspannung gegen Wasser zwischen 20 und 30 mN/m auf.

Die mittelpolare Lipidphase enthält vorteilhaft Lipide, die gewählt werden aus der Gruppe der Dialkylether, der Gruppe der gesättigten oder ungesättigten, verzweigten oder unverzweigten Alkohole. Es ist insbesondere vorteilhaft, wenn die Ölphase einen Gehalt an C12-15-Alkylbenzoat aufweist.

Ferner kann die Ölphase vorteilhaft Lipide enthalten, die gewählt werden aus der Gruppe der Guerbetalkohole. Guerbetalkohole sind benannt nach Marcel Guerbet, der ihre Herstellung erstmalig beschrieb. Sie entstehen nach der Reaktionsgleichung 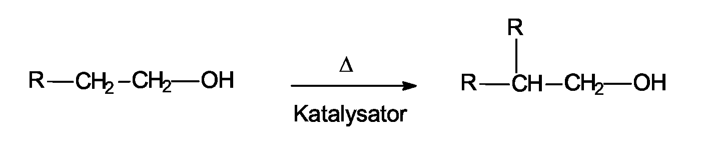 durch Oxidation eines Alkohols zu einem Aldehyd, durch Aldol-Kondensation des Aldehyds, Abspaltung von Wasser aus dem Aldol- und Hydrierung des Allylaldehyds. Guerbetalkohole sind selbst bei niederen Temperaturen flüssig und bewirken praktisch keine Hautreizungen. Vorteilhaft können sie als fettende, überfettende und auch rückfettend wirkende Bestandteile in Haut- und Haarpflegemitteln eingesetzt werden.

Die Verwendung von Guerbet-Alkoholen in Kosmetika ist an sich bekannt. Solche Species zeichnen sich dann meistens durch die Struktur 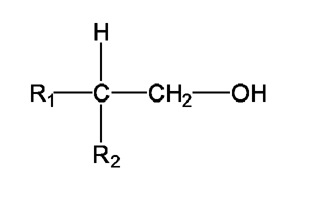 aus. Dabei bedeuten R1 und R2 in der Regel unverzweigte Alkylreste.

Erfindungsgemäß vorteilhaft werden der oder die Guerbet-Alkohole gewählt aus der Gruppe, bei denen
R1 = Propyl, Butyl, Pentyl, Hexyl, Heptyl oder Octyl und
R2 = Hexyl, Heptyl, Octyl, Nonyl, Decyl, Undecyl, Dodecyl, Tridecyl oder Tetradecyl.

Erfindungsgemäß bevorzugte Guerbet-Alkohole sind das 2-Butyloctanol - es hat die chemische Struktur 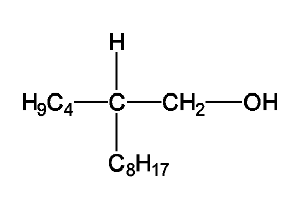 und ist beispielsweise unter der Handelsbezeichnung Isofol® 12 erhältlich - und das 2-Hexyldecanol - es hat die chemische Struktur 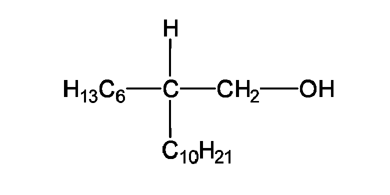 und ist beispielsweise unter der Handelsbezeichnung Isofol® 16 erhältlich. Auch Mischungen von Guerbet-Alkoholen sind vorteilhaft zu verwenden. Mischungen aus 2-Butyloctanol und 2-Hexyldecanol sind beispielsweise unter der Handelsbezeichnung Isofol® 14 erhältlich.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als Bestandteil der Ölphase einzusetzen.

Besonders vorteilhafte mittelpolare Lipide im Sinne der vorliegenden Erfindung sind die im folgenden aufgelisteten Substanzen:

| Handelsname | INCI-Name | Polarität [mN/m] |
|---|---|---|
| DUB VCI 10 | Isodecyl Neopentanoate | 29,9 |
| Dermol IHD | Isohexyldecanoate | 29,7 |
| Dermol 108 | Isodecyl Octanoate | 29,6 |
| Dihexyl Ether | Dihexyl Ether | 29,2 |
| Dermol 109 | Isodecyl 3,5,5 Trimethyl Hexanoate | 29,1 |
| Cetiol SN | Cetearyl Isononanoate | 28,6 |
| Isopropylpalmitat | Isopropylpalmitat | 28,8 |
| Jojobaöl Gold | | 26,2 |
| Wacker AK 100 | Dimethicone | 26,9 |
| Dermol 98 | 2- Ethylhexanosäure 3,5,5 Trimethylester | 26,2 |
| Eutanol G | Octyldodecanol | 24,8 |
| Isofol 16 | Hexyl Decanol | 24,3 |
| Dermol 139 | Isotridecyl 3,5,5 Trimethylhexanonanoate | 24,5 |
| Cetiol PGL | Hexyldecanol (+) Hexyl Decyl Laurate | 24,3 |
| Cegesoft C24 | Octyl Palmitate | 23,1 |
| M.O.D. | Octyldodeceyl Myristate | 22,1 |
| Macadamia Nut Oil | | 22,1 |
| Silikonöl VP 1120 | Phenyl Trimethicone | 22,7 |
| Isocarb 12 | Butyl Octanoicacid | 22,1 |
| Isopropylstearat | Isopropyl Stearate | 21,9 |
| Finsolv TN | C12-15 Alkyl Benzoate | 21,8 |
| Dermofeel BGC | Butylene Glycol Caprylate/Caprate | 21,5 |
| Miglyol 812 | Caprylic/Capric Triglyceride | 21,3 |
| Trivent OCG | Tricaprylin | 20,2 |
| Dermol 866 | PEG "Diethylhexanoate/ Diisononanoate/ Ethylhexyl Isononanoate | 20,1 |

Als mittelpolare Lipide werden insbesondere Lipide aus der Gruppe der Triglyceride, wie z. B. Caprylic Capric, sowie Guerbetalkohole wie z. B. Octyldodecanol, eingesetzt.

Darüberhinaus eignen sich die Lipide Isodecyl Neopentanoate, Isopropylpalmitat und Isopropylstearate sowie C12-15 Alkyl Benzoate.

Der Anteil der in der Formulierung eingesetzten mittelpolaren Lipide beträgt in der Summe 5 bis 20 Gew.-%, bevorzugt 7 bis 15 Gew.-% bezogen auf die Gesamtmasse der Gesamtlipidphase.

Bevorzugt sind Emulsion, die sowohl mittelpolare als auch polare Lipide umfassen.

Der Anteil an polaren Lipiden umfasst dabei mindestens 25 Gew.-%, bevorzugt 40 Gew.-%, bezogen auf das Gesamtgewicht der Lipidphase der Emulsion.

Bevorzugt werden Cocoglycerides (polar) sowie Caprylic/Capric Triglyceride, Octyldodecanol sowie Isopropylpalmitat gegebenenfalls in Kombination mit Dimethicone (mittelpolar), da diese Lipide in Kombination aufgrund ihrer physikochemischen Eigenschaften ein auf der Haut sensorisch ausgewogenes Profil ergeben.

Die Verwendung der 1, 2 Alkandiolen mit 4 bis 8 C-Atomen mit einem Ig p_{OW} von -1 bis +3, vorteilhaft von -1 bis +1, in Kombination mit polaren und/oder mittelpolaren Lipiden ermöglicht erstmals die Bereitstellung langzeitstabiler Emulsionen, die überwiegend eine polare Lipidphase enthalten ohne das dabei die kosmetische Formulierung sensorisch negativ beeinflusst wird. Langzeitstabil bedeutet 6 Monate bei 40°C und dadurch impliziert 2,5 Jahre Lagerstabilität bei Raumtemperatur.

Die Verwendung der erfindungsgemäßen Emulsion zur Herstellung langzeitstabiler kosmetischer Zubereitungen, die überwiegend eine polare Lipidphase enthalten, d.h. mehr als 40 Gew.-% an polaren bzw. mittelpoaren Lipiden, bevorzugt polare Lipide, ist damit erstmals möglich geworden.

Sensorische Daten werden standardmäßig im Sensory Research Panel (SRU-Panel) erhoben. Polare Lipidkomponenten wie z. B. Avocadoöl haben aufgrund ihrer Reichhaltigkeit eine hohe Schutz-und Pflegefunktion, die einerseits erwünscht ist aber sich negativ bei der Formulierung auf die Emulsionsstabilität auswirkt. So treten mitunter Ölabscheidung und/oder Wasserabscheidung auf. Durch die Hinzunahme von 1,2- Alkandiolen kann zum einen die Stabilität deutlich verbessert werden (6 Monate bei 40°C) und die sensorische Anmutung der hergestellten Emulsionen besitzt ein ausgewogeneres sensorisches Profil, d. h. ein verbessertes Einzugsvermögen und Verteilbarkeit bei gleich bleibender Schutz- und Pflegeleistung.

Erfindungsgemäße kosmetische und dermatologische Zubereitungen können in verschiedenen Formen vorliegen. So können sie z. B. eine Emulsion oder Mikroemulsion vom Typ Wasser-in-Öl (W/O), eine multiple Emulsion, beispielsweise vom Typ Wasser-in-Öl-in-Wasser (W/O/W), eine Emulsion vom Typ Öl-in-Wasser (O/W) oder eine Gelemulsion bzw. Hydrodispersionsgel darstellen. Erfindungsgemäß besonders vorteilhaft sind kosmetische Zubereitungen in Form von Öl-in-Wasser (O/W).

Es ist dabei bevorzugt, wenn für die erfindungsgemäße Emulsion als O/W-Emulgatoren Glycerylstearatcitrat (CAS 39175-72-9, INCI Glyceryl Stearate Citrate, z. B. Imwitor 370 der Firma Hüls), Polyglycerylmethylglucosedistearat (INCI Polyglyceryl Methyl Glucose Distearate, z. B. Tego Care 450 der Firma Goldschmidt) und/oder Polyethylenglycol(2000)monostearat (INCI PEG-40 stearate) eingesetzt werden.

Die wässrige Phase der emulsionsbasierenden Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder - monoethylether und analoge Produkte, ferner Alkohole niedriger C-Zahl, z. B. Ethanol, Isopropanol, 1,2-Propandiol, Glycerin.

Ein besonderer Vorzug der vorliegenden Erfindung ist es, dass sie gestattet, hohe Anteile an Polyolen, insbesondere Glycerin, d. h. 3 bis 30 Gew.-%, vorzugsweise 5 bis 25 Gew.-% und insbesondere bevorzugt 7,5 bis 15 Gew.-%, bezogen auf die Gesamtmasse der Zubereitung, einzusetzen.

Bevorzugt enthalten Emulsionen gemäß der vorliegenden Idee ein oder mehrere Hydrokolloide.

Weiterer Vorteil von kosmetischen Zubereitungen enthaltend kosmetische oder dermatologische Wirkstoffe, bevorzugt Antioxidantien, ist, dass sie die Haut vor oxidativer, photochemischer und/oder radikalischer Beanspruchung schützen können.

Weitere besonders vorteilhafte Wirkstoffe im Sinne der vorliegenden Erfindung sind natürliche Wirkstoffe und/oder deren Derivate, wie z. B. alpha-Liponsäure und deren Derivate, Phytoen, Ursolsäure, Sericoside, D-Biotin, Coenzym Q10 (Ubiquinon bzw. Ubiquinol und dessen Derivate), alpha-Glucosylrutin, Carnitin, Carnosin, natürliche und/oder synthetische Isoflavonoide, Kreatin, Taurin und/oder β-Alanin sowie 8-Hexadecen-1,16-dicarbonsäure (Dioic acid, CAS-Nummer 20701-68-2; vorläufige INCI-Bezeichnung Octadecendioic acid).

Auch ist die Verwendung der erfindungsgemäßen kosmetischen Zubereitungen zur Prophylaxe und Behandlung der Symptome von Altershaut, zur Verhinderung und Verminderung der Entstehung und Ausbreitung von Fältchen und Falten sowie zur Behandlung und Pflege gealterter Haut ist erfindungsgemäß. Geeignete Wirkstoffe für diesen Einsatzzweck sind: Ubichinon, Ubichinol, Retinol oder deren Derivate, Dehydroepiandrosteron (DHEA), Isoflavonoide, insbesondere Genistein oder Daidzein, Creatin, Phytoöstrogene, Niacinamid und/oder Polyphenole (alpha-Glucosylrutin).

Weiterhin ist die Verwendung der erfindungsgemäßen kosmetischen Zubereitungen zur Prophylaxe und Behandlung der Symptome trockener Haut bevorzugt. Geeignete Wirkstoffe für diesen Einsatzzweck sind: natürliche Öle (Sonnenblumen-, Nachtkerzensamen-, Jojoba-, Macadamiaöl, Rhizinusöl), Ceramide, insbesondere Ceramid I, III und VI, Cholesterin, Phytosterole, Carnitin und seine Derivate, Harnstoff, Polyole wie Glycerin, Pseudoceramide und Taurin, Fettalkohole sowie Wachse.

Es ist dem Fachmann natürlich bekannt, dass kosmetische Zubereitungen zumeist nicht ohne die üblichen Hilfs- und Zusatzstoffe denkbar sind. Die kosmetischen und dermatologischen Zubereitungen können dementsprechend ferner kosmetische Hilfsstoffe enthalten, wie sie üblicherweise in solchen Zubereitungen verwendet werden, beispielsweise Konsistenzgeber, Filmbildner, Stabilisatoren, Füllstoffe, Konservierungsmittel, Parfüme, Substanzen zum Verhindern des Schäumens, Farbstoffe, Pigmente, die färbende Wirkung haben, Verdickungsmittel, oberflächenaktive Substanzen, Emulgatoren, weich machende, anfeuchtende und/oder feuchthaltende Substanzen, entzündungshemmende Substanzen, zusätzliche Wirkstoffe wie Vitamine oder Proteine, Lichtschutzmittel, Insektenrepellentien, Bakterizide, Viruzide, Wasser, antimikrobiell, proteolytisch oder keratolytisch wirksame Substanzen, oder andere übliche Bestandteile einer kosmetischen oder dermatologischen Formulierung wie Alkohole, Polyole, Polymere, Schaumstabilisatoren und/oder organische Lösungsmittel.

Ebenso vorteilhaft ist es die erfindungsgemäßen Zubereitungen als Sonnenschutzmittel zu verwenden. Dementsprechend enthalten die Zubereitungen im Sinne der vorliegenden Erfindung vorzugsweise mindestens eine UV-A-, UV-B- und/oder Breitbandfiltersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Weitere vorteilhafte Substanzen zur Pflege der Haut oder des Haares sind beispielsweise anfeuchtende bzw. feuchthaltende Mittel (so genannte Moisturizer). Im Sinne der vorliegenden Idee werden daher vorteilhaft zugesetzt Substanzen gewählt aus der Gruppe Glycerin, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 erhältlich ist.

Es ist gegebenenfalls möglich und vorteilhaft, die erfindungsgemäßen Zusammensetzungen als Grundlage für pharmazeutische Formulierungen zu verwenden.

Erfindungsgemäß vorteilhafte Darreichungsformen der erfindungsgemäßen Zubereitungen sind Cremes, Salben, Hydrodispersionen, Lotionen, Tinkturen, Pumpsprays, Aerosolsprays, wässrige Lösungen, Reinigungssubstrate und dergleichen.

Bevorzugte Verwendung ist eine Darreichungsform als Creme, Lotion oder Pumpspray, eingeschränkt auch als Gesichtswasser.

Die erfindungsgemäßen kosmetischen Zubereitungen zeigen ein sensorisch ausgewogenes Profil auf der Haut, d. h. ein verbessertes Einzugsvermögen und Verteilbarkeit bei gleich bleibender Schutz- und Pflegeleistung als vergleichbare Emulsionen des Satndes der Technik (SRU-Panel)

In den nachfolgenden Beispielen sind verschiedenen erfindungsgemäßen Zubereitungen und deren Verwendung dargestellt. Die angegebenen Anteilswerte sind Gewichtsprozente bezogen auf die Gesamtmasse der Zubereitung, sofern nichts anderes angegeben ist:

### Beispiele

| Beispiel | 1 Creme | 2 Lotion | 3 Lotion | 4 Creme | 5 Creme | 6 Creme | 7 Lotion | 8 Spray |
|---|---|---|---|---|---|---|---|---|
| Glyceryl Stearate Citrat | | 1,0 | | 2,0 | | | | 2,0 |
| PEG-40 Stearat | 1,0 | | | | | 1,5 | | |
| Polyglycerylmethylglucosedistearat | | | 2,0 | | 3,0 | | 1,5 | 0,5 |
| Glycerylstearate | 1,5 | | | | | 1,5 | | |
| Cetyl Alkohol | | 0,5 | | 2,0 | 1,5 | 0,75 | 1,0 | |
| Stearyl Alcohol | | 0,5 | 0,5 | | | 0,75 | | |
| Cetearyl Alkohol | 2,0 | | | | | | | 1,5 |
| Caprylic/Capric Triglycerid | 5,0 | 4,0 | | 5,0 | | 6,0 | 3,0 | 0,5 |
| Ethylhexylcocoat | | | 2,0 | | | | 1,0 | |
| Octyldodecanol | | 1,0 | 3,0 | | 5,0 | | 2,0 | |
| Dibutyl Adipate | 2,0 | | 1,0 | 2,0 | | | 3,5 | |
| Cyclomethicon | 2,0 | | | | | 3,0 | | |
| Dimethicon | 1,0 | | | | | | | 1,5 |
| Dicaprylyl Carbonat | | 2,0 | | | 2,0 | | | 3,5 |
| Cocoglycerides | | 3,0 | 1,5 | | 2,0 | 5,0 | | 1,0 |
| Natürliche Öle (wie z. B. Jojobaöl / Sonnenblumenöl) | 1,5 | | 3,0 | 0,5 | 1,0 | | 2,0 | 2,5 |
| 1,2 Hexandiol | 0,5 | 0,75 | 2,0 | 0,3 | 1,0 | 1,2 | 0,5 | 0,75 |
| Trisodium EDTA | 0,2 | 0,1 | | 0,05 | | 0,1 | 0,3 | |
| Iminodisuccinat | 0,1 | | 0,1 | | 0,3 | | | 0,5 |
| Phenoxyethanol | 0,3 | 0,1 | | 0,5 | 0,7 | | | 0,4 |
| Parabene | 0,4 | | 0,3 | | 0,3 | | 0,2 | |
| Hexamidin Diisethionat | | 0,1 | | | 0,05 | | 0,1 | |
| Imidodiazolidinyl Urea | | | | | | 0,2 | | 0,2 |
| DMDM Hydantoin | | | 0,2 | | | 0,1 | | |
| lodopropynyl Butylcarbamat | | | | 0,2 | | | 0,05 | |
| | | | | | | | | |
| Glycerin | 10,0 | 3,0 | 7,0 | 8,0 | 15,0 | 20,0 | 0,5 | 2,0 |
| Tocopherylacetat | 0,2 | 0,5 | 0,75 | | | 0,3 | | 1,0 |
| Alcohol Denat. | 5,0 | | 2,5 | | | 7,5 | | 7,5 |

## Patentansprüche

1. Emulsion enthaltend über 40 Gew.-% ein oder mehrerer polarer und/oder mittelpolarer Lipide, bezogen auf das Gesamtgewicht der Lipidphase der Emulsion, mit einer Grenzflächenspannung gegen Wasser von weniger als 30 mN/m und ein oder mehrere 1,2 - Alkandiole mit 4 bis 8 C-Atomen und einem Ig p_{OW} im Bereich von - 1 bis + 3.

2. Emulsion nach Anspruch 1, **dadurch gekennzeichnet, dass** die Alkandiole eine Ig p_{OW} im Bereich von - 1 bis + 1 aufweisen.

3. Emulsion nach Anspruch 1 oder 2, **dadurch gekennzeichnet, dass** als Alkandiol 1,2-Hexandiol gewählt wird.

4. Emulsion nach einem der Ansprüche 1 oder 2, **dadurch gekennzeichnet, dass** als Alkandiole die Kombinationen von 1, 2 Hexandiol mit Methylpropandiol und/oder Ethylhexylglycerin gewählt wird.

5. Emulsion nach einem der vorstehenden Ansprüche, **dadurch gekennzeichnet, dass** über 40 Gew.-% ein oder mehrerer polarer Lipide, bezogen auf das Gesamtgewicht der Lipidphase der Emulsion, mit einer Grenzflächenspannung gegen Wasser von weniger als 20 mN/m gewählt werden.

6. Emulsion nach einem der vorstehenden Ansprüche 1 bis 4 enthaltend mittelpolare und polare Lipide.

7. Emulsion nach Anspruch 6, **dadurch gekennzeichnet, dass** der Anteil an polaren Lipiden mindestens 25 Gew.-%, bevorzugt mindestens 40 Gew.-%, bezogen auf das Gesamtgewicht der Lipidphase der Emulsion, beträgt.

8. Emulsion nach einem der Ansprüche 1 bis 7, **dadurch gekennzeichnet, dass** die Lipide gewählt werden aus der Gruppe Cocoglycerides, Caprylic/Capric Triglyceride, Octyldodecanol und/oder Isopropylpalmitat.

9. Emulsion nach Anspruch 8, **dadurch gekennzeichnet, dass** die Lipide in Kombination mit Dimethicone gewählt werden.

10. Emulsion nach einem der vorstehenden Ansprüche, **dadurch gekennzeichnet, dass** es sich um eine O/W-Emulsion handelt.

11. Emulsion nach Anspruch 10, **dadurch gekennzeichnet, dass** als O/W-Emulgatoren Glycerylstearatcitrat, Polyglycerylmethylglucosedistearat und/oder Polyethylenglycol(2000)monostearat eingesetzt werden.

12. Verwendung einer Emulsion nach einem der vorstehenden Ansprüche als kosmetische Zubereitung.

13. Verwendung der Emulsion nach Anspruch 12 in Form einer Creme, Lotion oder Pumpspray.

14. Verwendung der Emulsion nach einem der Ansprüche 1 bis 11 zur Herstellung langzeitstabiler kosmetischer Zubereitungen, die überwiegend eine polare Lipidphase enthalten.
